# EUROPÄISCHE PATENTSCHRIFT

(11) **EP 0 551 927 B1**
(45) Veröffentlichungstag und Bekanntmachung des Hinweises auf die Patenterteilung: **23.09.1998**
(21) Anmeldenummer: 93102949.0
(22) Anmeldetag: 03.04.1985
(51) Int. Cl.: A61K 38/55, C07K 5/06

(54) **Verfahren zur Behandlung der Herzinsuffizienz**
Method for the treatment of cardiac insufficiency
Méthode de traitement de l'insuffisance cardiaque

(30) Priorität: 12.04.1984 DE 3413710
(43) Veröffentlichungstag der Anmeldung: 21.07.1993
(62) Teilanmeldung aus: 85104028.7
(73) Patentinhaber: HOECHST AKTIENGESELLSCHAFT, 65926 Frankfurt am Main (DE)
(72) Erfinder: Henning, Rainer, Dr., W-6234 Hattersheim/M. (DE); Urbach, Hansjörg, Dr., W-6242 Kronberg/Ts. (DE); Teetz, Volker, Dr., W-6238 Hofheim/Ts. (DE); Geiger, Rolf, Prof. Dr., Verstorben (DE); Schölkens, Bernward, Prof. Dr., W-6233 Kelkheim/Ts. (DE)

(56) Entgegenhaltungen:
- CHEMICAL ABSTRACTS, vol. 100, no. 19, 7. Mai 1984, Columbus, Ohio, US; abstract no. 150853c, J. XIANG ET AL 'Influence of the novel non-sulfhydryl converting enzyme inhibitor on isolated rat hearts.' Seite 34 ;

## Beschreibung

In EP-A 49658 werden substituierte Iminodiazide zur Behandlung des Bluthochdrucks oder der Herzinsuffizienz beschrieben.

Die Erfindung betrifft ein Verfahren zur Behandlung der Herzinsuffizienz durch perorale oder parenterale Anwendung der Angiotensin-Converting-Enzym-Inhibitoren
N-(1-S-Carbethoxy-3-phenyl-propyl)-S-alanyl-S-1,2,3,4-tetra-hydroisochinolin-3-carbonsäure
N-(1-S-Carbethoxy-3-phenyl-propyl)-S-alanyl-(2S,3aR,7aS)-octahydroindol-2-carbonsäure
N-(1-S-Carboxy-3-phenyl-propyl)-S-alanyl-(2S,3aR,7aS)-octahydroindol-2-carbonsäure
oder deren physiologisch verträglichen Salze.

Diese Verbindungen lassen sich z.B. noch dem in der deutschen Patentanmeldung P 33 33 455.2 beschriebenen Verfahren herstellen, in dem die in der Anmeldung beschriebenen tert. Butyl- oder Benzylreste in bekannter Weise durch saure oder alkalische Hydrolyse oder durch Edelmetall-katalysierte Hydrogenolyse in die Monocarbonsäurederivate überführt werden. Die oben aufgeführten Verbindungen lassen sich leicht mit physiologisch verträglichen Säuren oder Basen (im Falle von Mono- oder Dicarbonsäuren) in die entsprechenden Salze (z.B. Hydrochloride, Maleinate, Fumarate, etc.) überführen und als Salze die erfindungsgemäße Verwendung finden.

Die oben aufgeführten Verbindungen sind Hemmstoffe des Angiotensin Converting Enzymes (ACE) und können auch zur Bekämpfung des Bluthochdrucks verschiedener Genese eingesetzt werden. Die oben aufgeführten Verbindungen sind bekannt aus E-PA-46953, und, E-PA-84164.

In Ausübung der erfindungsgemäßen Methode können die oben beschriebenen Angiotensin Converting Enzyme-Inhibitoren an Säugern wie Affen, Hunden, Katzen, Ratten, Menschen etc. angewendet werden. Die für die erfindungsgemäße Verwendung geeigneten Verbindungen werden zweckmäßig in üblicher Weise in pharmazeutische Präparate eingearbeitet. Sie können in die üblichen Verabreichungsformen, wie Kapseln, Tabletten, Dragees, Lösungen, Salben, Emulsionen und auch in Depot-Form gebracht werden. Der Wirkstoff kann gegebenenfalls auch in mikroverkapselter Form vorliegen. Die Präparate können verträgliche, organische bzw. anorganische Begleitstoffe, beispielsweise Granulierstoffe, Klebe- und Bindemittel, Gleitmittel, Suspendiermittel, Lösungsmittel, antibakterielle Mittel, Netzmittel und Konservierungsmittel enthalten. Orale und parenterale Anwendungsformen werden bevorzugt. Die oben aufgeführten Verbindungen können in Dosierungen von 0,1 - 50 mg pro Dosis ein- bis dreimal täglich verabreicht werden.

Die Wirksamkeit der oben aufgeführten Verbindungen bei verschiedenen Formen der Herzinsuffizienz kann aus ihrer Wirkung in verschiedenen Testmodellen abgeleitet werden. Als Beispiele sollen im folgenden jeweils die Ergebnisse mit N-(1-S-Carbethoxy-3-phenyl-propyl)-S-alanyl-cis-endo-2-azabicyclo[3,3,0]octan-3-S-carbonsäure (Formel II) dienen.

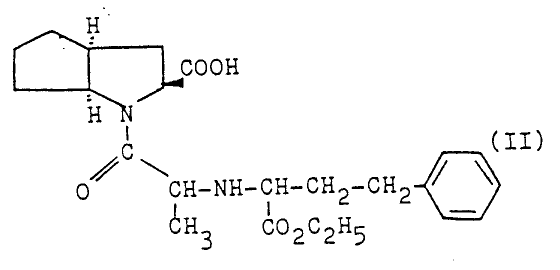

Es ist bekannt, daß Converting-Enzyme-Inhibitoren das Enzym nicht nur im Serum, sondern auch in verschiedenen Geweben, besonders auch in Herzen zu hemmen vermögen. Diese Hemmung im Herzgewebe verringert die Bildung von Angiotensin II und damit dessen nachteilige Einflüsse auf verschiedene Parameter der Herztätigkeit.

Orale Behandlung an spontan hypertonen Ratten mit einer einmaligen Dosis von 1 mg/kg hemmt das ACE im Herzen länger als 24 Stunden signifikant. Dagegen wird mit 30 mg/kg N-(1-Carbethoxy-3-phenyl-propyl)-S-alanyl-S-prolin (Enalapril) per os bereits nach mehr als 6 Stunden keine signifikante Hemmung mehr beobachtet. Chronische orale Behandlung von spontan hypertensiven Ratten über zwei Wochen mit 1 mg/kg der Verbindung der Formel II führt am Ende der Behandlung zu einer 55 %igen Hemmung des ACE im Herzen, mit 30 mg/kg Enalapril per os wird dagegen unter sonst gleichen Bedingungen keine Hemmung beobachtet. Zusätzlich führt die systemische Blutdrucksenkung, die durch die oben aufgeführten Verbindungen hervorgerufen wird, zu einer Nachlastsenkung und damit zu einer Entlastung des insuffizienten Herzens.

Postganglionäre sympathische Stimulation am isolierten Herzen führt über gesteigerte Catecholamin-Freisetzung zum Anstieg von Herzfrequenz, Kontraktionskraft und zu einem Abfall der Coronardurchströmung. Orale Vorbehandlung mit 1 mg/kg per os der Verbindung der Formel II an Kaninchen vermindert signifikant den Einfluß auf Herzfrequenz und Coronardurchströmung, läßt jedoch die Kontraktionskraft unbeeinflußt. Diese Effekte wirken zum Schutz des insuffizienten Herzens zusammen.

Angiotensin I vermindert, Bradykinin dagegen verstärkt die Coronardurchströmung am isolierten Herzen von Kaninchen, Meerschweinchen und Ratten. Orale Vorbehandlung der Tiere mit 1 mg/kg der Verbindung der Formel II peroral vermindert den Effekt von Angiotensin I und steigert die Wirkung von Bradykinin signifikant. Ein gleicher Effekt kann erst mit 30 mg/kg Enalapril erreicht werden.

Die folgenden Beispiele geben die Anwendungsformen zur Behandlung der Herzinsuffizienz nach der erfindungsgemäßen Methode an. Die oben aufgeführten Verbindungen können analog den Beispielen in die entsprechenden Anwendungsformen gebracht werden.

### Beispiel 1

Herstellung des erfindungsgemäß verwendeten Mittels zur oralen Anwendung in der Behandlung der Herzinsuffizenz.

1000 Tabletten, die je 10 mg 1-N-(1-S-Carbethoxy-3-phenyl-propyl)-S-alanyl-1S,3S,5S-2-azabicyclo[3.3.0]-octan-3-carbonsäure enthalten, werden mit den folgenden Hilfsmitteln hergestellt:

| | |
|---|---|
| N-(1-S-Carbethoxy-3-phenyl-propyl)-S-alanyl-1S,3S,5S-2-azabicyclo[3.3.0]octan-3-carbonsäure | 10 g |
| Maisstärke | 140 g |
| Gelatine | 7,5 g |
| Mikrokristalline Cellulose | 2,5 g |
| Magnesiumstearat | 2,5 g |

N-(1-S-Carbethoxy-3-phenyl-propyl)-S-alanyl-1S,3S-5S,-2-azabicyclo[3.3.0]octan-3-carbonsäure und Maisstärke werden mit einer wäßrigen Gelatine-Lösung gemischt. Die Mischung wird getrocknet und zu einem Granulat vermahlen. Mikrokristalline Cellulose und Magnesiumstearat werden mit dem Granulat vermischt. Das entstandene Granulat wird zu 1000 Tabletten gepreßt, wobei jede Tablette 10 mg des ACE-Hemmers enthält.

Diese Tabletten können zur Behandlung der Herzinsuffizenz verwendet werden.

### Beispiel 2

Analog Beispiel 1 werden 1000 Tabletten hergestellt, die je 10 mg N-(1-S-Carbethoxy-3-phenyl-propyl)-S-alanyl-(2S,3aR,7aS)-octahydroindol-2-carbonsäure-hydrochlorid enthalten.

### Beispiel 3

Die Herstellung einer Injektionslösung zur Behandlung der Herzinfuffizenz wird im folgenden beschrieben:

| | |
|---|---|
| N-(1-S-Carbethoxy-3-phenyl-propyl)-S-alanyl-1S,3S,5S-2-azabicyclo[3.3.0]octan-3-carbonsäure | 250 mg |
| Methylparaben | 5 g |
| Propylparaben | 1 g |
| Natriumchlorid | 25 g |
| Wasser für Injektion | 5 l |

N-(1-S-Carbethoxy-3-phenyl-propyl)-S-alanyl-1S,3S,5S-2-azabicyclo[3.3.0]octan-3-carbonsäure, die Konservierungsstoffe und Natriumchlorid werden in 3 l Wasser für Injektion gelöst und auf 5 l mit Wasser für Injektion aufgefüllt. Die Lösung wird steril gefiltert und aseptisch in vorsterilisierte Flaschen gefüllt, die mit sterilisierten Gummikappen verschlossen werden. Jede Flasche enthält 5 ml Lösung.

### Beispiel 4

Tabletten, die zur Behandlung der Herzinsuffizenz verwendet werden können, werden wie in Beispiel 1 beschrieben hergestellt, mit der Ausnahme, daß anstelle von N-(1-S-Carbethoxy-3-phenyl-propyl)-S-alanyl-1S,3S,5S-2-azabicyclo[3.3.0]octan-3S-carbonsäure
N-(1-S-Carbethoxy-3-phenyl-propyl)-S-alanyl-2S,3aR,7aS-octahydroindol-2-carbonsäure angewendet wird.

### Beispiel 5

Eine Injektionslösung wird analog der in Beispiel 3 beschriebenen Vorschrift hergestellt, mit der Ausnahme, daß anstelle von N-(1-S-Carbethoxy-3-phenyl-propyl)-S-alanyl-1S,3S,5S-2-azabicyclo[3.3.0]octan-3-carbonsäure
N-(1-S-Carbethoxy-3-phenyl-propyl)-S-alanyl-2S,3aR,7aS-octahydroindol-2-carbonsäurehydrochlorid oder
N-(1-S-Carbethoxy-3-phenyl-propyl)-S-alanyl-2S,3aR,7aS-octahydroindol-2-carbonsäure angewendet werden.

## Patentansprüche

1. Verwendung des Angiotensin-Converting-Enzym-Inhibitors N-(1-S-Carbethoxy-3-phenyl-propyl)-S-alanyl-(2S,3aR,7aS)-octahydroindol-2-carbonsäure oder dessen physiologisch verträglichen Salzes bei der Herstellung eines Arzneimittels zur Behandlung der Herzinsuffizienz.

2. Verwendung des Angiotensin-Converting-Enzym-Inhibitors N-(1-S-Carboxy-3-phenyl-propyl)-S-alanyl-(2S,3aR,7aS)-octahydroindol-2-carbonsäure oder ein physiologisch verträgliches Salz davon bei der Herstellung eines Arzneimittels zur Behandlung der Herzinsuffizienz.

3. Verwendung des Angiotensin-Converting-Enzym-Inhibitors N-(1-S-Carbethoxy-3-phenyl-propyl)-S-alanyl-S-1,2,3,4-tetrahydroisochinolin-3-carbonsäure oder ein physiologisch verträgliches Salz davon bei der Herstellung eines Arzneimittels zur Behandlung der Herzinsuffizienz.

4. Verwendung gemäß Anspruch 1-3, dadurch gekennzeichnet, daß die Angiotensin-Converting-Enzym-Inhibitoren oral oder parenteral angewendet werden.

5. Verwendung gemäß Anspruch 1-3, dadurch gekennzeichnet, daß die Angiotensin-Converting-Enzym-Inhibitoren für die entsprechenden Anwendungsformen mit pharmazeutisch geeigneten Trägerstoffen bzw. Hilfsstoffen kombiniert werden.

## Claims

1. The use of the angiotensin-converting enzyme inhibitor N-(1-S-carbethoxy-3-phenylpropyl)-S-alanyl-(2S,3aR,7aS)-octahydroindole-2-carboxylic acid or its physiologically tolerated salt in the preparation of a pharmaceutical for treating cardiac insufficiency.

2. The use of the angiotensin-converting enzyme inhibitor N-(1-S-carbethoxy-3-phenylpropyl)-S-alanyl-(2S,3aR,7aS)-octahydroindole-2-carboxylic acid or a physiologically tolerated salt thereof in the preparation of a pharmaceutical for treating cardiac insufficiency.

3. The use of the angiotensin-converting enzyme inhibitor N-(1-S-carbethoxy-3-phenylpropyl)-S-alanyl-S-1,2,3,4-tetrahydroisoquinoline-3-carboxylic acid or a physiologically tolerated salt thereof in the preparation of a pharmaceutical for treating cardiac insufficiency.

4. The use as claimed in claims 1-3, wherein the angiotensin-converting enzyme inhibitors are employed orally or parenterally.

5. The use as claimed in claims 1-3, wherein the angiotensin-converting enzyme inhibitors are combined with pharmaceutically appropriate vehicles or auxiliary substances for the corresponding use forms.

## Revendications

1. Utilisation de l'inhibiteur de l'enzyme de conversion de l'angiotensine acide N-(1-S-carbéthoxy-3-phénylpropyl)-S-alanyl-(2S,3aR,7aS)-octahydro-indole-2-carboxylique ou d'un sel physiologiquement acceptable de celui-ci, dans la fabrication d'un médicament destiné au traitement de l'insuffisance cardiaque.

2. Utilisation de l'inhibiteur de l'enzyme de conversion de l'angiotensine acide N-(1-S-carboxy-3-phényl-propyl)-S-alanyl-(2S,3aR,7aS)-octahydro-indole-2-carboxylique ou d'un sel physiologiquement acceptable de celui-ci, dans la fabrication d'un médicament destiné au traitement de l'insuffisance cardiaque.

3. Utilisation de l'inhibiteur de l'enzyme de conversion de l'angiotensine acide N-(1-S-carbéthoxy-3-phényl-propyl)-S-alanyl-S-1,2,3,4-tétrahydro-isoquinoléine-3-carboxylique ou d'un sel physiologiquement acceptable de celui-ci, dans la fabrication d'un médicament destiné au traitement de l'insuffisance cardiaque.

4. Utilisation selon l'une des revendications 1 à 3, caractérisée en ce que les inhibiteurs de l'enzyme de conversion de l'angiotensine sont administrés par voie orale ou parentérale.

5. Utilisation selon la revendication 1, caractérisée en ce que, pour les formes d'administration correspondantes, on associe les inhibiteurs de l'enzyme de conversion de l'angiotensine à des véhicules ou adjuvants pharmaceutiques appropriés.
